# EUROPÄISCHE PATENTSCHRIFT

(11) **EP 0 669 100 B1**
(45) Veröffentlichungstag und Bekanntmachung des Hinweises auf die Patenterteilung: **18.11.1998**
(21) Anmeldenummer: 95890038.3
(22) Anmeldetag: 22.02.1995
(51) Int. Cl.: A61B 17/00, A61M 35/00

(54) **Vorrichtung zur Applikation eines Mehrkomponenten-Gewebeklebstoffes**
Application device for multi-component tissue adhesive
Dispositif pour appliquer une colle de tissu à plusieurs composant

(30) Priorität: 28.02.1994 AT 414/94
(43) Veröffentlichungstag der Anmeldung: 30.08.1995
(73) Patentinhaber: IMMUNO Aktiengesellschaft, A-1221 Wien (AT)
(72) Erfinder: Linder, Albert, Dr. med. Dipl.-phys., D-71229 Leonberg - Höfingen (DE); Kellner, Andreas, Ing., A-2122 Ulrichskirchen (AT); Habison, Georg, Dr., A-1040 Wien (AT); Zülow, Günther, Dr., A-1090 Wien (AT)
(74) Vertreter: Weinzinger, Arnulf, Dipl.-Ing.

(56) Entgegenhaltungen:
- EP-A- 0 105 493
- EP-A- 0 315 222
- DE-A- 4 223 356
- US-A- 4 179 068

## Beschreibung

Die Erfindung betrifft eine Vorrichtung zur Applikation eines Mehrkomponenten-Gewebeklebstoffes, mit in einem einstückigen Bauteil von einem Anschlußteil zu einem Abgabeteil verlaufenden Komponenten-Förderkanälen für die Komponenten des Gewebeklebstoffes sowie einem Gas-Förderkanal für ein zur Zerstäubung der Gewebeklebstoff-Komponenten dienendes medizinisches Gas, wobei alle Förderkanäle in gesonderten Austrittsöffnungen an einer Stirnseite des Abgabeteils münden. Eine derartige Vorrichtung ist bekannt aus der EP-A-315222.

Ein Gewebeklebstoff wird zumeist als Mehrkomponentenmaterial zur Verfügung gestellt, welches bei der Anwendung gemischt wird. Durch die Reaktion der Komponenten (üblicherweise einerseits eine Fibrinogenlösung und andererseits eine Thrombinlösung) kommt es zur Verfestigung des Gewebeklebstoffes und damit zu einem erwünschten Verbinden von Gewebe- oder Organteilen, zur Wundversiegelung, Blutstillung und dergleichen. Für die Applikation eines Gewebeklebstoffes ist es daher erforderlich, die einzelnen Gewebeklebstoff-Komponenten zunächst strikt getrennt voneinander aufzubewahren und dann in weiterer Folge möglichst gleichzeitig zu vermischen und zu applizieren. Die Applikation kann dabei auch die Verwendung eines medizinischen Gases zum Zerstäuben eines Gewebeklebstoffes umfassen. Mit einer solchen Zerstäubung soll einerseits die Homogenität der Mischung gewährleistet werden, und andererseits kann der Gewebeklebstoff auch großflächig aufgetragen werden. Besonders bevorzugte Anwendungsgebiete sind die Blutstillung, Wundversiegelung, bei Verbrennungen, Hauttransplantationen und das Aussprühen von Körperhöhlen. Die Behandlung von Resektionsflächen parenchymatöser Organe erfolgt vorzugsweise ebenfalls durch Sprühapplikation. Die Größe der Auftragfläche ist normalerweise durch den jeweiligen Abstand des Gerätes von der Auftragungsstelle variierbar.

Aus der EP-B-37 393 bzw. der korrespondierenden US-A-4 359 049 ist eine Vorrichtung zur Applikation eines Gewebeklebstoffes bekannt, die mit einem Sprühkopf ausgebildet werden kann. Der Sprühkopf ist gleichzeitig als Sammelkopf ausgeführt, durch den die beiden Gewebeklebstoff-Komponenten aus einzelnen Spritzenkörpern über getrennte Förderkanäle bis zu Austrittsöffnungen geführt werden. Der Sprühkopf weist weiters einen Förderkanal für ein steriles Gas auf, der sich innerhalb des Kopfes in zwei Äste teilt, deren Austrittsöffnungen im Bereich der Mündungen der Komponenten-Förderkanäle angeordnet sind. Die Achsen dieser Austrittsöffnungen sind etwa rechtwinkelig zur Austrittsrichtung der beiden Gewebeklebstoff-Komponenten gerichtet. Dabei werden zwei gesonderte Sprühkegel gebildet, die sich in einem Abstand vom Sprühkopf schneiden bzw. vereinigen, wobei ein komplettes Mischen der versprühten Komponenten erst ab einem Mindestabstand möglich ist.

Eine in vielen Aspekten ähnliche Vorrichtung wird von der Behringwerke AG, Marburg, Deutschland, unter der Bezeichnung "Berijet"® vertrieben. Dabei ist ein flacher einstückiger Sammelkopf-Sprühkopf-Bauteil vorgesehen, von dem ein Gas-Anschlußteil im rechten Winkel absteht. Von diesem Gas-Anschlußteil führt im Inneren ein Gas-Förderkanal zu einer Stirnseite, wobei dort der Gas-Förderkanal durch eine stirnseitig vorspringende Trennwand in zwei gesonderte Teilkanäle unterteilt ist, die getrennt voneinander an der Stirnseite - beidseits der vorspringenden Trennwand - ausmünden; in jeden dieser Teilkanäle mündet vor deren Austrittsöffnungen ein entsprechender Förderkanal für eine der zwei Komponenten des zu applizierenden Gewebeklebstoffes schräg ein. Auch hier werden somit zwei Gasstrahlen gebildet, die sich nach ihrem gesonderten Austreten vereinigen, damit sich die in ihnen mitgeführten Komponenten mischen. Auch hier kann es jedoch zu einer ungenügenden Mischung kommen.

Eine weitere Vorrichtung zur Applikation eines Gewebeklebstoffes ist in der AT-B-379 311 bzw. der korrespondierenden EP-A-156 098 und EP-A-315 222 bzw. der US-A-4 631 055 beschrieben. Hier weist ebenfalls ein Sammel- bzw. Sprühkopf einen Förderkanal für ein medizinisches Gas auf, der eng benachbart und spitzwinkelig zu Förderkanälen für die beiden Komponenten eines Gewebeklebstoffes an die Stirnseite eines Aufsteck-Fortsatzes des Kopfes führt. Auf diesen Aufsteck-Fortsatz wird insbesondere ein gesonderter, passender, mehrlumiger Katheter in fluchtender Verbindung seiner Lumina mit den Förderkanälen aufgesetzt. Als Alternative hierzu kann eine Mischkanüle aufgesetzt werden, in der die einzelnen Komponenten im Gasstrom gemischt werden, wozu überdies insbesondere eine die Turbulenz der durchströmenden Komponenten fördernde Innenfläche vorgesehen wird, was aber mit einem erhöhten Herstellungsaufwand verbunden ist.

In Ann Thorac Surg, 55, 1593-600 (1993), S.1595-1596, ist ebenfalls ein Gerät mit gesondertem Katheter beschrieben, welches das Versprühen eines Gewebeklebstoffes bei thorakoskopischen Operationen erlaubt. Die beiden Komponenten des Gewebeklebstoffes werden über einzelne Spritzenkörper zu einem Verbindungskopf geführt, der voneinander getrennte Förderkanäle für die beiden Komponenten aufweist, und an dem der Katheter befestigt ist. Der Katheter besteht aus einem Stahlrohr, das innenseitig so ausgebildet ist, daß zwei Wegwerf-Kunststoffrohre, jeweils eines für eine Komponente des Gewebeklebstoffes, eingeschoben werden können. Die Mündungen der Kunststoffrohre stehen über die Mündung des Stahlrohres vor. Das Stahlrohr weist dann zwei Kanäle für die Komponenten des Gewebeklebstoffes auf, die jeder für sich von einem Hohlraum umgeben sind, durch den Stickstoffgas geleitet wird. Das Gas wird dem Katheter über ein an ihm vorgesehenes Anschlußstück nahe dem Verbindungskopf zugeführt.

Ein ähnliches Gerät ist in General Thoracic Surgery, 42-48 (1993), für die Thorakoskopie beschrieben. Hier kommt ebenfalls ein Stahlrohr zur Anwendung, das einen Katheter aus Kunststoff zum Fördern der beiden Komponenten des Gewebeklebstoffes aufnimmt. Der Kunststoffkatheter ist am distalen Ende des Stahlrohres durch Vorsprünge zentriert. Somit ist der Kunststoffkatheter in einem Hohlraum angeordnet, durch welchen ein Gas geleitet wird. Die Zufuhr des Gases erfolgt ebenfalls direkt zum Katheter über ein entsprechendes Anschlußstück.

Bei den beiden letzterwähnten Geräten mit Stahlrohr-Katheter ist nachteilig, daß das Stahlrohr den Anschluß für das medizinische Gas aufweist, was jedoch bei der chirurgischen Anwendung hinderlich ist; ein Katheter sollte an sich keine sperrigen Teile aufweisen, um Verletzungen zu vermeiden.

Es ist nun Ziel der Erfindung, eine Vorrichtung der eingangs angeführten Art zu schaffen, die nicht nur einfach in der Herstellung und handlich bei der Benützung ist, sondern insbesondere auch beim Zerstäuben eine gute, homogene Mischung der Komponenten eines Gewebeklebstoffes sicherstellt.

Die erfindungsgemäße Vorrichtung der eingangs erwähnten Art ist dadurch gekennzeichnet, daß die Komponenten-Förderkanäle gemeinsam zumindest auf dem Teil ihrer Länge unmittelbar vor den Austrittsöffnungen vom Gas-Förderkanal umschlossen sind. Vorzugsweise verlaufen dabei alle Förderkanäle zumindest auf dem Teil ihrer Länge unmittelbar vor den Austrittsöffnungen zueinander parallel.

Bei einer derartigen Ausbildung wird eine besonders homogene Mischung der Gewebeklebstoff-Komponenten im Sprühstrahl erreicht, wobei von Vorteil ist, daß die durch die Austrittsöffnungen ihrer Förderkanäle austretenden Komponenten allseitig von einem einzigen sie ringförmig umgebenden Gasstrahl umschlossen sind, welcher die Komponenten mitreißt und verwirbelt, so daß sie gut vermischt werden. Dabei kann, je nach Geometrie der Austrittsöffnungen an der Stirnseite der Vorrichtung, auch ein außerordentlich enger Sprühstrahl oder aber ein divergierender Sprühkegel erreicht werden, wobei es im letzteren Fall zweckmäßig ist, die ringförmige Austrittsöffnung des Gas-Förderkanals an ihrer radial äußeren Seite mit einer leicht divergierenden Begrenzungsfläche auszubilden. Die vorliegende Vorrichtung ist auch besonders vorteilhaft bei der Benützung, wobei die Handhabung bei der Vorbereitung einer Gewebeklebstoff-Applikation ebenso einfach und bequem ist wie die Sprüh-Aufbringung des Gewebeklebstoffs selbst.

An sich könnte die Austrittsöffnung des Gas-Förderkanals sowie der daran anschließende Teil dieses Gas-Förderkanals eine beliebige geschlossene Querschnittsform, etwa die Form einer flachen Ellipse, aufweisen, jedoch hat es sich im Interesse einer problemlosen, von einer bestimmten Ausrichtung beim Sprühen unabhängigen Handhabung der vorliegenden Vorrichtung als günstig erwiesen, wenn der Gas-Förderkanal, zumindest auf dem die Komponenten-Förderkanäle umschließenden Teil seiner Länge, als im Querschnitt kreisförmiger Ringkanal ausgebildet ist.

Eine besonders vorteilhafte Ausführungsform der erfindungsgemäßen Vorrichtung ist dadurch gekennzeichnet, daß die Austrittsöffnungen der Komponenten-Förderkanäle äußerstenfalls in der Ebene der Austrittsöffnung des Gas-Förderkanals, vorzugsweise relativ zu dieser zurückversetzt, angeordnet sind. Bei einer solchen Ausbildung wird eine homogene Mischung der Gewebeklebstoff-Komponenten auch dann gewährleistet, wenn bei der Benützung die Vorrichtung sehr nahe zur Applikationsstelle gehalten wird.

Um den Sprühkegel variieren zu können, ist es von besonderem Vorteil, wenn am Abgabeteil ein den Gas-Förderkanal außen begrenzender, gesonderter Begrenzungsteil in Richtung der Förderkanäle längsverstellbar angeordnet ist. Der Begrenzungsteil kann dabei über eine Schraub- oder Steckverbindung mit der übrigen Vorrichtung verstellbar verbunden sein, und er ermöglicht es, bei einem vorgegebenen, bestimmten Abstand zur Applikationsstelle durch seine Verstellung je nach Bedarf eine kleinflächige, praktisch punktförmige, oder eine großflächige Auftragung des Gewebeklebstoffes vorzunehmen. Im Interesse einer einfachen Ausbildung und Herstellung ist es dabei günstig, wenn der Begrenzungsteil hülsenförmig ausgebildet ist. Weiters ist es für die Variation des Sprühkegels von Vorteil, wenn sich die Innenfläche des Begrenzungsteils im Bereich benachbart der vorderen Stirnseite zu dieser hin, z.B. konisch, erweitert.

Die vorliegende Vorrichtung ermöglicht weiters in vorteilhafter Weise die Integration eines Katheters, um den Gewebeklebstoff auch an schwer zugänglichen Stellen aufbringen zu können. Demgemäß ist eine andere vorteilhafte Ausführungsform der erfindungsgemäßen Vorrichtung dadurch gekennzeichnet, daß der Abgabeteil durch einen mit der übrigen Vorrichtung einstückigen, mehrlumigen Katheter gebildet ist, der für jede Gewebeklebstoff-Komponente ein Lumen als Förderkanal aufweist, und daß weiters der mehrlumige Katheter ein diese Komponenten-Förderkanäle umschließendes Lumen als Gas-Förderkanal enthält. Um eine gleichmäßige Gaszuführung und somit eine gleichmäßige Applikation der Gewebeklebstoff-Komponenten sicherzustellen, ist es dabei weiters von Vorteil, wenn der Katheter innerhalb des Gas-Förderkanal-Lumens zumindest im Bereich nahe den Austrittsöffnungen radiale Haltestege enthält, die die Komponenten-Förderkanäle innerhalb des sie umschließenden Gas-Förderkanals zentriert halten und gegebenenfalls in Abstand vor der vorderen Stirnseite enden. Der Katheter ist ferner vorzugsweise flexibel, d.h. biegsam, ausgeführt bzw. plastisch vorformbar, was durch entsprechende Materialwahl für den die vorliegende Vorrichtung bildenden einstückigen Bauteil sichergestellt werden kann; in diesem Fall kann es weiters zweckmäßig sein, wenn der Katheter ein zusätzliches Lumen für einen Formdraht enthält. Mit diesem Formdraht kann dem Katheter die bei der Applikation an einer schwer zugänglichen Körperstelle erforderliche Form vorgegeben werden.

Der die Vorrichtung bildende Bauteil einschließlich gegebenenfalls des Katheters wird insbesondere aus einem eine Biegsamkeit des Katheters gewährleistenden Kunststoff hergestellt. Vorzugsweise hat ein flexibler Katheter einen Außendurchmesser kleiner als 2,5 mm.

Der Katheter eignet sich aber auch zur minimal invasiven chirurgischen Anwendung, wenn er von einer starren Hülse (Adapter) umschlossen ist oder selbst starr ausgebildet ist, wodurch das Einführen des Katheters in einen Trokar und somit z.B. in eine Bauchhöhle erleichtert wird. Der Außendurchmesser eines starren Katheters beträgt insbesondere 4-6 mm, vorzugsweise etwa 5 mm, und paßt damit zum Innendurchmesser eines handelsüblichen Trokars.

Bei der vorliegenden Vorrichtung wird das medizinische Gas, das zum Zerstäuben oder Versprühen der Gewebeklebstoff-Komponenten zugeführt wird, direkt in einem hinteren Anschlußteil der Vorrichtung zugeführt, wobei die Vorrichtung gleichzeitig als Verbindungsstück von Spritzenkörpern dienen kann. Der Anschlußteil für die Zuführung des Gases ist dabei vorzugsweise ein schräg, d.h. in einem spitzen Winkel zum proximalen Ende der Vorrichtung oder einem stumpfen Winkel zum Abgabeteil der Vorrichtung, angeordneter Anschlußstutzen- Damit wird der Vorteil erzielt, daß der Gas-Anschlußstutzen bei der Benützung dem Anwender wenig hinderlich ist. Als besonders vorteilhaft hat es sich in diesem Zusammenhang erwiesen, wenn der Anschlußstutzen des Gas-Förderkanals unter einem Winkel von 120° bis 150° zur Richtung der Förderkanäle im Abgabeteil angeordnet ist.

Die Erfindung wird nachstehend anhand von in der Zeichnung veranschaulichten bevorzugten Ausführungsbeispielen, auf die sie jedoch nicht beschränkt sein soll, noch weiter erläutert. Im einzelnen zeigen:
Fig.1 eine geschnittene Draufsicht auf eine Vorrichtung zur Applikation von Gewebeklebstoff;
Fig.2 einen Längsschnitt durch diese Vorrichtung gemäß der Linie II-II in Fig.1;
Fig.3 eine Stirnansicht dieser Vorrichtung, gemäß Pfeil III in Fig.1;
Fig.3A in einer Ansicht ähnlich Fig.3 eine demgegenüber etwas modifizierte Ausführungsform der Vorrichtung;
Fig.4 in einer Schnittdarstellung ähnlich Fig.1 eine abgewandelte, zumindest derzeit besonders bevorzugte Ausführungsform der erfindungsgemäßen Vorrichtung, mit einem längsverstellbaren hülsenförmigen Begrenzungsteil zur Einstellung des abgegebenen Sprühkegels;
Fig.5 eine zugehörige Stirnansicht des Abgabeteils dieser Vorrichtung gemäß Fig.4;
die Fig.6 und 7 in Darstellungen ähnlich jenen von Fig.4 und 5 diese Vorrichtung mit einer anderen Einstellung des Begrenzungsteils, zur Erzielung einer anderen Sprühkegel-Einstellung;
Fig.8 in einer teilweise unterbrochenen Querschnittsdarstellung ähnlich Fig.1, Fig.4 und Fig.6 eine weitere Ausführungsform der erfindungsgemäßen Vorrichtung, mit integriertem Sprühkatheter;
Fig.9 eine zugehörige Stirnansicht dieser Vorrichtung, ähnlich der Darstellung in Fig.3; und
Fig.9A in einer vergleichsweisen Stirnansicht eine Darstellung einer gegenüber Fig.9 geringfügig modifizierten Ausführungsform, mit einem zusätzlichen Lumen im Katheterteil der Vorrichtung für die Aufnahme eines Formdrahts.

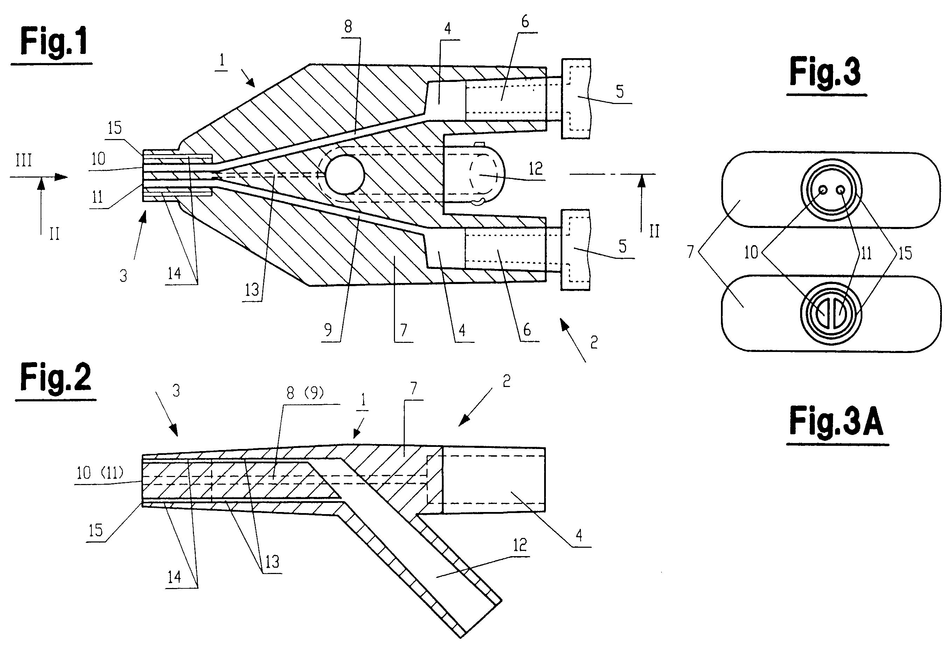

Bei allen in der Zeichnung veranschaulichten Ausführungsformen der vorliegenden Vorrichtung ist ein einstückiger Bauteil 1, insbesondere aus Kunststoff, mit einem Anschlußteil 2 für die Zuführung von Gewebeklebstoff-Komponenten (üblicherweise eine Fibrinogenlösung und ein Thrombinlösung) sowie für die Zuführung eines zur Zerstäubung verwendeten medizinischen Gases (üblicherweise sterile Druckluft, Stickstoff oder Kohlendioxid), sowie mit einem Abgabeteil 3 vorhanden. Der Anschlußteil 2 enthält zur Zuführung der Gewebeklebstoff-Komponenten zwei Einsteckkonusse 4 (vorzugsweise Luer-Konusse), in die Einweg-Spritzenkörper 5, die die Klebstoffkomponenten enthalten, mit Hilfe von passend ausgebildeten Anschlußkonussen 6 angeschlossen werden. Die Einsteckkonusse 4 setzen sich innerhalb eines Sammelkopfes 7 der Vorrichtung in Komponenten-Förderkanälen 8, 9 fort, die von diesen Einsteckkonussen 4 zu stirnseitigen Austrittsöffnungen 10, 11 im Abgabeteil 3 führen. Die Komponenten-Förderkanäle 8, 9 zur Führung der Gewebeklebstoff-Komponenten können dabei im Querschnitt kreisförmig, wie in Fig.3 gezeigt, oder aber auch halbkreisförmig, wie aus Fig.3A ersichtlich, ausgebildet sein, und zwar zumindest im Abgabeteil 3 nahe der Stirnseite mit den Austrittsöffnungen 10, 11.

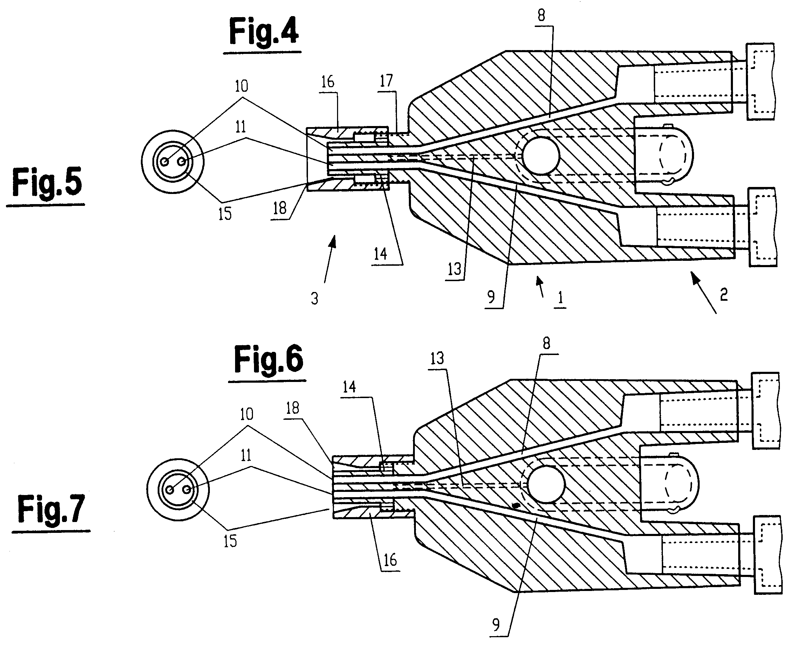

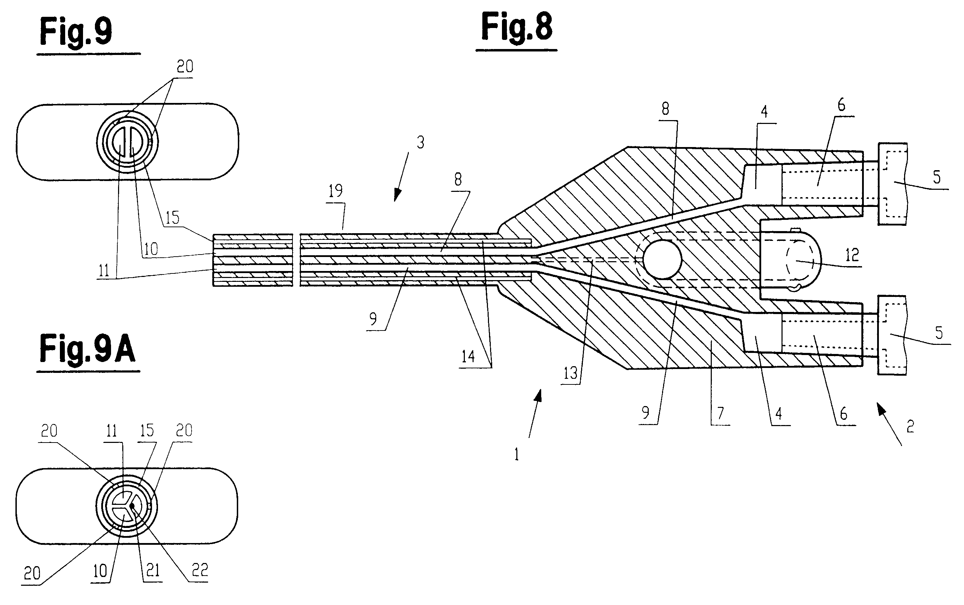

Weiters ist an der Unterseite des Sammelkopfes 7, der im wesentlichen durch den flachen, allgemein plattenförmigen Bauteil 1 gebildet ist und zugleich in der Ausführungsform gemäß Fig.1 bis 5 einen Sprühkopf bildet, im Anschlußteil 2 ein Anschlußstutzen 12 für ein medizinisches Gas, z.B. Stickstoff, sterile Druckluft oder Kohlendioxid, angebracht, und zwar insbesondere in einem stumpfen Winkel von 120° bis 150° zur Längsebene des Sammelkopfes 7 an der Abgabeseite. Von diesem Gas-Anschlußstutzen 12 führt ein oder führen mehrere Gas-Förderkanäle 13 in Richtung zur Stirnseite des Sprühkopfes 7 hin, wobei er bzw. sie im Abgabeteil 3 in einen ringförmig geschlossenen Kanal 14, mit einer vorzugsweise kreisringförmigen Austrittsöffnung 15 (s. Fig.3, Fig.3A), übergeht bzw. übergehen. Der ringförmige Gas-Förderkanal-Teil 14 umschließt dabei ringförmig die Komponenten-Förderkanäle 8, 9 im Bereich von deren Austrittsöffnungen 10, 11, wie sich insbesondere aus den verschiedenen Stirnansichten in der beiliegenden Zeichnung z.B. in Fig.3, 3A aber auch Fig.5, 7 wie auch Fig.9 bzw. 9A, besonders deutlich erkennen läßt. Im Abgabeteil 3 sind daher alle Kanäle 8, 9 und 14 zueinander parallel.

Wird nun im Betrieb ein Gasfluß über den Anschlußstutzen 12 und den oder die Förderkanäle 13, 14 zur Austrittsöffnung 15 freigegeben, so werden die beiden Gewebeklebstoff-Komponenten, die durch Drücken der Spritzenkolben den Komponenten-Förderkanälen 8, 9 zugeführt werden, und die an den Austrittsöffnungen 10, 11 austreten, vom an der Austrittsöffnung 15 austretenden Gasstrom erfaßt und in einem homogenen Sprühkegel unter guter Durchmischung zerstäubt.

Die Ausführungsform gemäß Fig.4 bis 7 entspricht weitgehend jener gemäß Fig.1 bis 3, mit Ausnahme einer verstellbaren Austrittsöffnung für das medizinische Gas, und demgemäß soll sich die Beschreibung dieser Ausführungsform nachfolgend im wesentlichen auf die Unterschiede im Vergleich zur Ausführungsform gemäß Fig.1 bis 3 beschränken.

Im einzelnen münden die Komponenten-Förderkanäle 8, 9 in der vorstehend beschriebenen Weise stirnseitig in den Austritts-Öffnungen 10, 11 aus, die beispielsweise wiederum kreisförmig im Ouerschnitt ausgebildet ein können. Der an den Gas-Förderkanal 13 anschließende ringförmige Gas-Förderkanal-Teil 14 wird nunmehr jedoch, anders als in der Ausführungsform gemäß Fig.1 bis 3, anstatt durch einen starren Außenwandteil des Abgabeteils 3 durch einen gesonderten, hülsenförmig ausgebildeten, längsverstellbar angeordneten Außenwand-Begrenzungsteil 16 begrenzt. Dieser Begrenzungsteil 16 kann beispielsweise auf ein zylindrisches Ansatzstück 17 des Bauteils 1 aufgeschraubt oder verschiebbar aufgesteckt sein, wozu entsprechende Außen- bzw. Innengewinde oder aber entsprechende, ein Abfallen des Begrenzungsteiles 16 verhindernde Rastnoppen oder dergl. (nicht gezeigt) vorgesehen sein können. In Fig.4 und 6 sind die für eine Schraubverbindung vorgesehen Außen- und Innengewinde schematisch angedeutet.

An ihrem der Stirnseite benachbarten vorderen Teil weist die Innenfläche des hülsenförmigen Begrenzungsteils 16 eine sich konisch erweiternde Form auf, wie dies in Fig.4 bei 18 gezeigt ist. Durch axiales Verstellen des hülsenförmigen Begrenzungsteiles 16 kann demgemäß, zufolge Variation der die Austrittsöffnungen 10, 11 der Komponenten-Förderkanäle 8, 9 unmittelbar umgebenen Austrittsöffnung 15 des Gas-Förderkanals 13, 14, der Sprühkegel in seinem Auffächerungswinkel variiert werden. So wird in der Position gemäß Fig.4, in der der Begrenzungsteil 16 in Längsrichtung so weit wie möglich vorgeschoben wurde, eine schmale Austrittsöffnung 15 und somit ein schmaler Sprühkegel erreicht. In der Position gemäß Fig.6 ist der Begrenzungsteil 16 so weit wie möglich zurückgeschoben, wobei eine breite Austrittsöffnung 15 für das Gas gebildet wird und somit ein breiter Sprühkegel herbeigeführt werden kann. In Zwischenpositionen des Begrenzungsteils 16 werden entsprechende Zwischeneinstellungen für den Sprühkegel erreicht.

Auch in der Ausführungsform gemäß Fig.8 und 9 entspricht die dargestellte Vorrichtung weitgehend, was den Sammelkopf 7 anlangt, den bisher beschriebenen Ausführungsformen, so daß sich auch hier eine diesbezügliche neuerliche Beschreibung erübrigen kann. Bei der Ausführungsform gemäß Fig.8 und 9 setzen sich jedoch die verschiedenen Förderkanäle 8, 9 (für die Gewebeklebstoff-Komponenten) sowie 13 (für das medizinische Gas) in entsprechenden Kanälen in einem mit dem Sammelkopf 7 einstückigen, mehrlumigen Katheter 19 fort, der hier den Abgabeteil 3 bildet. Dabei sind im ringförmigen Gas-Förderkanalteil 14 (Druckluftspalt) ungefähr radiale Haltestege 20 vorgesehen, die die Ringform des Kanals 14 im Katheter 19 aufrechterhalten, d.h. den inneren Katheterteil mit den Komponenten-Förderkanälen 8, 9 zentriert halten. Diese Stege 20 enden kurz vor den Austrittsöffnungen 10, 11, 15, damit der Gasauslaß an der Stirnseite des Katheters 19 wieder durchgehend geschlossen ringförmig, insbesondere kreisrund, sein kann.

Durch entsprechende Materialwahl für den die Vorrichtung bildenden Bauteil 1 (Sammelkopf 7 einschließlich Katheter 19) kann der Katheter 19 entweder starr oder aber flexibel ausgeführt werden.

Sofern der Katheter 19 flexibel ausgeführt wird, hat er vorzugsweise einen Durchmesser kleiner 2,5 mm, und er kann weiters, wie in Fig.9A gezeigt ist, ein zusätzliches Lumen 21 für einen Formdraht 22 enthalten. Dadurch kann der flexible Katheter 19 in eine gewünschte Form gebracht werden, die er dann beibehält.

Wird die Ausführungsform mit Katheter 19 zur Verwendung in der minimal invasiven Chirurgie vorgesehen, so wird vorzugsweise das Katheterrohr 19 starr ausgebildet, und es wird ein Außendurchmesser von 5 mm gewählt; andererseits kann hierfür der Katheter 19 mit Hilfe eines über ihn geschobenen Adapterrohres (nicht gezeigt) mit geeignetem Durchmesser über ein Zugangsinstrument eingeführt werden.

Wenn die Erfindung vorstehend anhand von besonders bevorzugten Ausführungsbeispielen näher erläutert wurde, so sind doch selbstverständlich weitere Abwandlungen und Modifikationen im Rahmen der Erfindung möglich. So ist es beispielsweise denkbar, auch in der Ausführungsform mit integriertem Katheter 19 gemäß Fig.8 und 9 im Querschnitt kreisrunde Komponenten-Förderkanäle 8, 9, mit entsprechenden kreisrunden Austrittsöffnungen 10, 11, ähnlich wie in Fig.3 oder 5 dargestellt, vorzusehen. Andererseits ist es selbstverständlich auch denkbar, in der Ausführungsform gemäß Fig.4 und 6, mit der längsverstellbaren Begrenzungshülse 16 zur Einstellung des Sprühkegels, die Austrittsöffnungen 10, 11 sowie die daran anschließenden Teile der Komponenten-Förderkanäle 8, 9 im Querschnitt allgemein halbkreisförmig, ähnlich wie in Fig.3A oder 9 dargestellt zu gestalten. Der die Vorrichtung bildende Bauteil 1 (Sammelkopf/Sprühkopf 7 bzw. Sammelkopf 7 einschließlich Katheter 19) wird bevorzugt aus einem üblichen in der Medizin verwendeten Kunststoff, wie z.B. einen Polyolefin (Polyethylen, Polypropylen), Polyurethan, PVC oder ABS (Acrylnitrilbutadienstyrol), durch Spritzgießen bzw. Extrudieren hergestellt. In ähnlicher Weise kann selbstverständlich auch der hülsenförmige Begrenzungsteil 16 aus einem derartigen Kunststoffmaterial hergestellt werden.

## Patentansprüche

1. Vorrichtung zur Applikation eines Mehrkomponenten-Gewebeklebstoffes, mit in einem einstückigen Bauteil (1) von einem Anschlußteil (2) zu einem Abgabeteil (3) verlaufenden Komponenten-Förderkanälen (8, 9) für die Komponenten des Gewebeklebstoffes sowie einem Gas-Förderkanal (13, 14) für ein zur Zerstäubung der Gewebeklebstoff-Komponenten dienendes medizinisches Gas, wobei alle Förderkanäle (8, 9, 13, 14) in gesonderten Austrittsöffnungen (10, 11, 15) an einer Stirnseite des Abgabeteils (3) münden, dadurch gekennzeichnet, daß die Komponenten-Förderkanäle (8, 9) gemeinsam zumindest auf dem Teil ihrer Länge unmittelbar vor den Austrittsöffnungen (10, 11) vom Gas-Förderkanal (14) umschlossen sind.

2. Vorrichtung nach Anspruch 1, dadurch gekennzeichnet, daß alle Förderkanäle (8, 9, 13, 14) zumindest auf dem Teil ihrer Länge unmittelbar vor den Austrittsöffnungen (10, 11, 15) zueinander parallel verlaufen.

3. Vorrichtung nach Anspruch 1 oder 2, dadurch gekennzeichnet, daß der Gas-Förderkanal (14) zumindest auf dem die Komponenten-Förderkanäle (8, 9) umschließenden Teil seiner Länge als im Querschnitt kreisförmiger Ringkanal ausgebildet ist.

4. Vorrichtung nach einem der Ansprüche 1 bis 3, dadurch gekennzeichnet, daß die Austrittsöffnungen (10, 11) der Komponenten-Förderkanäle äußerstenfalls in der Ebene der Austrittsöffnung (15) des Gas-Förderkanals (13, 14), vorzugsweise relativ zu dieser zurückversetzt, angeordnet sind.

5. Vorrichtung nach einem der Ansprüche 1 bis 4, dadurch gekennzeichnet, daß am Abgabeteil (3) ein den Gas-Förderkanal (14) außen begrenzender, gesonderter Begrenzungsteil (16) in Richtung der Förderkanäle (8, 9, 14) längsverstellbar angeordnet ist.

6. Vorrichtung nach Anspruch 5, dadurch gekennzeichnet, daß der Begrenzungsteil (16) hülsenförmig ausgebildet ist.

7. Vorrichtung nach Anspruch 5 oder 6, dadurch gekennzeichnet, daß sich die Innenfläche (18) des Begrenzungsteils (16) im Bereich benachbart der vorderen Stirnseite zu dieser hin, z.B. konisch, erweitert.

8. Vorrichtung nach einem der Ansprüche 1 bis 4, dadurch gekennzeichnet, daß der Abgabeteil (3) durch einen mit der übrigen Vorrichtung (7) einstückigen, mehrlumigen Katheter (19) gebildet ist, der für jede Gewebeklebstoff-Komponente ein Lumen als Förderkanal (8, 9) aufweist, und daß weiters der mehrlumige Katheter (19) ein diese Komponenten-Förderkanäle (8, 9) umschließendes Lumen als Gas-Förderkanal (14) enthält.

9. Vorrichtung nach Anspruch 8, dadurch gekennzeichnet, daß der Katheter (19) innerhalb des Gas-Förderkanal-Lumens (14) zumindest im Bereich nahe den Austrittsöffnungen (10, 11, 15) radiale Haltestege (20) enthält, die die Komponenten-Förderkanäle (8, 9) innerhalb des sie umschließenden Gas-Förderkanals (14) zentriert halten und gegebenenfalls in Abstand vor der vorderen Stirnseite enden.

10. Vorrichtung nach Anspruch 8 oder 9, dadurch gekennzeichnet, daß der Katheter (19) biegsam bzw. vorformbar ausgeführt ist.

11. Vorrichtung nach Anspruch 10, dadurch gekennzeichnet, daß der Katheter (19) ein zusätzliches Lumen (21) für einen Formdraht (22) enthält.

12. Vorrichtung nach Anspruch 10 oder 11, dadurch gekennzeichnet, daß der Katheter (19) einen Außendurchmesser kleiner als 2,5 mm hat.

13. Vorrichtung nach Anspruch 8 oder 9, dadurch gekennzeichnet, daß der Katheter (19) starr ausgeführt ist.

14. Vorrichtung nach Anspruch 13, dadurch gekennzeichnet, daß der Katheter (19) einen Außendurchmesser von 4 mm bis 6 mm, vorzugsweise etwa 5 mm, hat.

15. Vorrichtung nach einem der Ansprüche 1 bis 14, dadurch gekennzeichnet, daß als Anschlußteil für den Gas-Förderkanal (13, 14) ein Anschlußstutzen (12) unter einem stumpfen Winkel zum Abgabeteil (3) angeordnet ist.

16. Vorrichtung nach Anspruch 15, dadurch gekennzeichnet, daß der Anschlußstutzen (12) des Gas-Förderkanals (13, 14) unter einem Winkel von 120° bis 150° zur Richtung der Förderkanäle (8, 9, 14) im Abgabeteil (3) angeordnet ist.

## Claims

1. An apparatus for applying a multiple component tissue adhesive comprising component conveying channels (8, 9) provided for the components of the tissue adhesive and extending in a one-piece structure part (1) from a connecting part (2) to a delivery part (3), as well as a gas conveying channel (13, 14) for a medicinal gas serving for atomizing the tissue adhesive components, all of the conveying channels (8, 9, 13, 14) ending in separate exit openings (10, 11, 15) at a front side of the delivery part (3), characterised in that the component conveying channels (8, 9) together are surrounded by the gas conveying channel (14) at least over that part of their longitudinal extension immediately upstream of their exit openings (10, 11).

2. An apparatus according to claim 1, characterised in that all the conveying channels (8, 9, 13, 14) extend parallel to each other at least over that part of their longitudinal extension immediately upstream of their exit openings (10, 11, 15).

3. An apparatus according to claim 1 or 2, characterised in that at least over that part of its longitudinal extension enclosing the component conveying channels (8, 9), the gas conveying channel (14) is designed as an annular channel of circular cross section.

4. An apparatus according to any one of claims 1 to 3, characterised in that the exit openings (10, 11) of the component conveying channels at the utmost are arranged in the plane of the exit openings (15) of the gas conveying channel (13, 14), and preferably are rearwardly offset relative to the same.

5. An apparatus according to any one of claims 1 to 4, characterised in that a separate delimiting part (16) outwardly delimiting the gas conveying channel (14) is arranged on the delivery part (3) so as to be longitudinally displaceable in the direction of the conveying channels (8, 9, 14).

6. An apparatus according to claim 5, characterised in that the delimiting part (16) is sleeve-shaped.

7. An apparatus according to claim 5 or 6, characterised in that the inner surface (18) of the delimiting part (16) is divergent, e.g. widens conically, in the region neighbouring the front end side, towards the same.

8. An apparatus according to any one of claims 1 to 4, characterised in that the delivery part (3) is formed by a multi-lumen catheter (19) formed in one piece with the remaining apparatus (7), which has a lumen as a conveying channel (8, 9) for each component of the tissue adhesive, and that furthermore, the multi-lumen catheter (19) comprises a lumen enclosing these component-conveying channels (8, 9) as a gas conveying channel (14).

9. An apparatus according to claim 8, characterised in that the catheter (19) at least in its region close to the exit openings (10, 11, 15) comprises radial holding webs (20) within the gas conveying channel lumen (14), which holding webs keep the component conveying channels (8, 9) centered within the enclosing gas conveying channel (14), and which optionally end at a distance in front of the front end side.

10. An apparatus according to claim 8 or 9, characterised in that the catheter (19) is flexible or pre-shapable.

11. An apparatus according to claim 10, characterised in that the catheter (19) contains an additional lumen (21) for a shaping wire (22).

12. An apparatus according to claim 10 or 11, characterised in that the catheter (19) has an external diameter of less than 2.5 mm.

13. An apparatus according to claim 8 or 9, characterised in that the catheter (19) is rigid.

14. An apparatus according to claim 13, characterised in that the catheter (19) has an external diameter of from 4 mm to 6 mm, preferably about 5 mm.

15. An apparatus according to any one of claims 1 to 14, characterised in that as connecting part for the gas conveying channel (13, 14), a connection piece (12) is arranged at an obtuse angle relative to the delivery part (3).

16. An apparatus according to claim 15, characterised in that the connection piece (12) of the gas conveying channel (13, 14) is arranged at an angle of from 120° to 150° relative to the direction of the conveying channels (8, 9, 14) in the delivery part (3).

## Revendications

1. Dispositif pour l'application d'un histoadhésif à plusieurs composants, avec des canaux de transport de composants (8, 9) pour les composants de l'histoadhésif qui s'étendent dans une partie constitutive d'une pièce (1) d'une partie de raccordement (2) à une partie d'émission (3) ainsi qu'un canal de transport de gaz (13, 14) pour un gaz médical servant à la pulvérisation des composants de l'histoadhésif, où tous les canaux de transport (8, 9, 13, 14) débouchent dans des ouvertures de sortie séparées (10, 11, 15) au niveau d'un côté frontal de la partie d'émission (3), caractérisé en ce que les canaux de transport de composants (8, 9) sont entourés ensemble au moins sur une partie de leur longueur juste avant les ouvertures de sortie (10, 11) par le canal de transport de gaz (14).

2. Dispositif selon la revendication 1, caractérisé en ce que tous les canaux de transport (8, 9, 13, 14) s'étendent parallèlement entre eux au moins sur une partie de leur longueur juste avant les ouvertures de sortie (10, 11, 15).

3. Dispositif selon la revendication 1 ou 2, caractérisé en ce que le canal de transport de gaz (14) est agencé sous forme d'un canal annulaire circulaire en section droite au moins sur la partie de sa longueur qui entoure les canaux de transport de composants (8, 9).

4. Dispositif selon l'une des revendications 1 à 3, caractérisé en ce que les ouvertures de sortie (10, 11) des canaux de transport de composants sont disposées dans le cas extrême dans le plan de l'ouverture de sortie (15) du canal de transport de gaz (13, 14), de préférence en étant en retrait par rapport à celui-ci.

5. Dispositif selon l'une des revendications 1 à 4, caractérisé en ce qu'une partie de limitation séparée (16) limitant extérieurement le canal de transport de gaz (14) est disposée de manière déplaçable longitudinalement dans la direction des canaux de transport (8, 9, 14) au niveau de la partie d'émission (3).

6. Dispositif selon la revendication 5, caractérisé en ce que la partie de limitation (16) est agencée sous forme de gaine.

7. Dispositif selon la revendication 5 ou 6, caractérisé en ce que la surface interne (18) de la partie de limitation (16) s'élargit dans le domaine voisin du côté frontal avant vers celui-ci, par exemple de manière conique.

8. Dispositif selon l'une des revendications 1 à 4, caractérisé en ce que la partie d'émission (3) est formée par un cathéter à plusieurs lumières (19) d'une pièce avec le reste du dispositif (7) qui comporte une lumière en tant que canal de transport (8, 9) pour chaque composant de l'histoadhésif, et en ce que, en outre, le cathéter à plusieurs lumières (19) contient une lumière entourant ces canaux de transport de composants (8, 9) en tant que canal de transport de gaz (14).

9. Dispositif selon la revendication 8, caractérisé en ce que le cathéter (19) contient à l'intérieur de la lumière formant canal de transport de gaz (14) au moins dans le domaine proche des ouvertures de sortie (10, 11, 15) des traverses de maintien radiales (20) qui maintiennent centrés les canaux de transport de composants (8, 9) à l'intérieur du canal de transport de gaz (14) qui les entoure et qui se terminent éventuellement à distance avant le côté frontal avant.

10. Dispositif selon la revendication 8 ou 9, caractérisé en ce que le cathéter (19) est agencé de manière flexible ou déformable.

11. Dispositif selon la revendication 10, caractérisé en ce que le cathéter (19) contient une lumière supplémentaire (21) pour un fil de forme (22).

12. Dispositif selon la revendication 10 ou 11, caractérisé en ce que le cathéter (19) a un diamètre externe inférieur à 2,5 mm.

13. Dispositif selon la revendication 8 ou 9, caractérisé en ce que le cathéter (19) est agencé de manière rigide.

14. Dispositif selon la revendication 13, caractérisé en ce que le cathéter (19) a un diamètre externe de 4 mm à 6 mm, de préférence d'environ 5 mm.

15. Dispositif selon l'une des revendications 1 à 14, caractérisé en ce qu'une tubulure de raccordement (12) est agencée sous un angle obtus avec la partie d'émission (3) en tant que partie de raccordement pour le canal de transport de gaz (13, 14).

16. Dispositif selon la revendication 15, caractérisé en ce que la tubulure de raccordement (12) du canal de transport de gaz (13, 14) est agencée sous un angle de 120° à 150° par rapport à la direction des canaux de transport (8, 9, 14) dans la partie d'émission (3).
